# EUROPEAN PATENT APPLICATION

(11) **EP 4 056 621 A1**
(43) Date of publication of application: **14.09.2022**
(21) Application number: 20884663.4
(22) Date of filing: 04.11.2020
(51) Int. Cl.: C08G 63/06

(54) **NOVEL POLYESTER DERIVED FROM BIOMASS**

(30) Priority: 06.11.2019 JP 2019201633
(71) Applicant: National Institute Of Advanced Industrial Science and Technology, Chiyoda-ku Tokyo 100-8921 (JP)
(72) Inventor: NEMOTO, Koji, Tsukuba-shi, Ibaraki 305-8560 (JP); YAMAMOTO, Atsushi, Tsukuba-shi, Ibaraki 305-8560 (JP); SATO, Kazuhiko, Tsukuba-shi, Ibaraki 305-8560 (JP)
(74) Representative: Gill Jennings & Every LLP
(86) International application number: PCT/JP2020/041166
(87) International publication number: WO 2021/090825

(57) **Abstract**

The present invention provides a biomass-derived polyester compound useful as a rubber material or an adhesive material, the compound including a multiple bond in the molecule. By synthesizing an ester compound composed of a hydroxycinnamic acid and a fatty acid, and carrying out polycondensation reaction using the ester compound as a monomer, a polyester compound containing cinnamic acid and a ricinoleic acid derivative that are alternately linked to each other can be efficiently obtained. For example, an ester compound of General Formula (1) is produced. (In the formula, the substituents R₁ and R₂ each represent a hydrogen atom or a methoxy group; the substituent R₃ represents an alkyl group or an alkenyl group; and n represents an integer of 10 to 100).

## Description

### TECHNICAL FIELD

The present invention relates to a novel polyester composed of a cinnamic acid derivative and a ricinoleic acid derivative.

### BACKGROUND ART

Compounds represented by cinnamic acid and ricinoleic acid are useful as chemical products that can be synthesized using a biomass as a raw material. The compounds are hopeful especially as polymer raw materials, and a variety of polyester compounds have been studied and developed as represented by Non-patent Document 1.

Polyester compounds composed of a cinnamic acid derivative, as represented by Non-patent Documents 2 and 3, are known to have high heat resistance (with a melting point of not less than 200°C) and crystallinity, but such compounds are poorly flexible, which is problematic. On the other hand, polyester compounds composed of a ricinoleic acid derivative, as represented by Non-patent Documents 4, have flexibility (they are oily compounds), but such compounds have low heat resistance, which is problematic.

### PRIOR ART DOCUMENTS

### [Non-patent Documents]

[Non-patent Document 1] Polymer Chemistry, 2014, 5, 3119-3141
[Non-patent Document 2] ACS Symposium Series, 2015, Vol. 1192, Chapter 24, pp. 401-409
[Non-patent Document 3] Green Chemistry, 2010, Vol. 12, pp. 1677-1872
[Non-patent Document 4] Biomacromolecules, 2015, Vol. 16, 2572-2587

### SUMMARY OF THE INVENTION

### PROBLEMS TO BE SOLVED BY THE INVENTION

As described above, for homopolymers composed only of a cinnamic acid derivative and homopolymers composed only of a ricinoleic acid derivative, simultaneous achievement of heat resistance and flexibility giving excellent processability has been difficult.

An object of the present invention is to provide, by controlling a polymer structure, a biomass-derived polyester compound extremely useful as a rubber material or an adhesive material, the compound comprising a multiple bond in the molecule.

### MEANS FOR SOLVING THE PROBLEMS

Recently, the present inventors discovered that, by synthesizing an ester compound composed of a cinnamic acid derivative and ricinoleic acid, and carrying out polycondensation reaction using the ester compound as a monomer, a copolymer containing a cinnamic acid derivative and a ricinoleic acid derivative that are alternately linked to each other can be efficiently obtained, thereby achieving the present invention based on this discovery.

The present invention relates to the following polyester compounds (1) to (6).
(1) A polyester compound comprising a copolymer containing a hydroxycinnamic acid and a fatty acid that are alternately directly linked to each other.
(2) The polyester compound according to claim 1, wherein the hydroxycinnamic acid is a compound represented by the following General Formula (2): 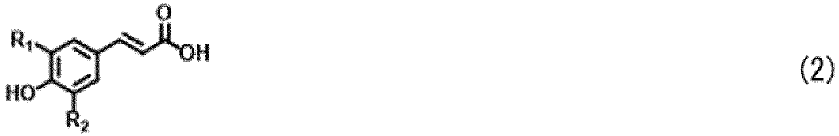 (wherein in the formula, the substituents R₁ and R₂ each represent a hydrogen atom or a methoxy group).
(3) The polyester compound according to claim 1 or 2, wherein the fatty acid is a compound represented by the following General Formula (3): 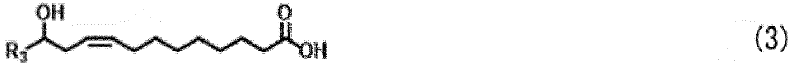 (wherein in the formula, the substituent R₃ represents an alkyl group or an alkenyl group).
(4) The polyester compound according to any one of claims 1 to 3, wherein the copolymer is represented by the following General Formula (1): 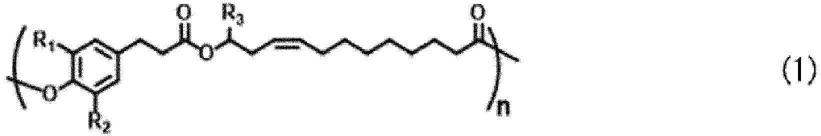 (wherein in the formula, the substituents R₁ and R₂ each represent a hydrogen atom or a methoxy group; R₃ represents an alkyl group or an alkenyl group; and n represents an integer of 10 to 100).
(5) The polyester compound according to any one of claims 1 to 4, wherein the hydroxycinnamic acid and/or the fatty acid is/are produced by fermentation using a biomass as a raw material, produced by hydrolysis reaction using a biomass as a raw material, or produced by extraction using a biomass as a raw material.
(6) The polyester compound according to any one of claims 1 to 5, further comprising a third component.

### EFFECT OF THE INVENTION

The novel polyester compound obtained by the present invention is extremely useful as a rubber material or an adhesive material. The compound has a regular structure, whose production is difficult for the random copolymerization method, which is a conventional standard means in the art.

Further, by the present invention, polyester compounds can be efficiently obtained using a variety of kinds of biomass-derived raw materials.

### MODE FOR CARRYING OUT THE INVENTION

The present invention relates to a polyester compound composed of a novel copolymer containing a hydroxycinnamic acid and a fatty acid that are alternately directly linked to each other, and a polyester compound comprising this copolymer.

The polyester compound comprising the copolymer means that the polyester compound further comprises a copolymer containing a third component.

Examples of such a copolymer include copolymers containing lactic acid, 3-hydroxybutanoic acid, 4-hydroxyvaleric acid, vanillic acid, or the like. The third component may be an optically active compound or a racemic body.

The hydroxycinnamic acid used is a compound represented by the following General Formula (2), and examples of the hydroxycinnamic acid include p-coumaric acid, ferulic acid, and sinapic acid. 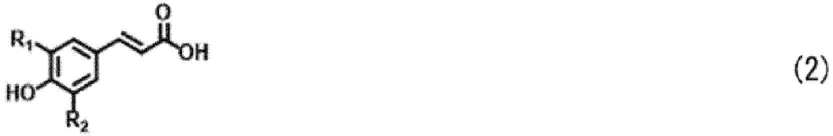 (In the formula, the substituents R₁ and R₂ each represent a hydrogen atom or a methoxy group.)

The hydroxycinnamic acid may be produced by fermentation using a biomass as a raw material, may be produced by hydrolysis reaction using a biomass as a raw material, or may be produced by extraction using a biomass as a raw material.

The fatty acid used is a compound represented by the following General Formula (3), and examples of the fatty acid include ricinoleic acid; and compounds in which a hydroxy group(s) is/are added to linoleic acid, oleic acid, linolenic acid, or the like. 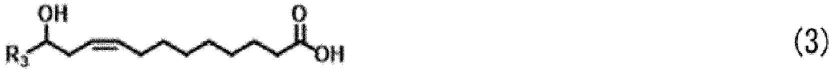 (In the formula, the substituent R₃ represents an alkyl group or an alkenyl group.)

The number of carbon atoms in the alkyl group or alkenyl group is 1 to 15, preferably 1 to 10. Examples of the substituent R₃ include alkyl groups such as C₆H₁₃.

The fatty acid used may be produced by fermentation using a biomass as a raw material, may be produced by hydrolysis reaction using a biomass as a raw material, or may be produced by extraction using a biomass as a raw material.

By synthesizing an ester compound composed of a hydroxycinnamic acid and a fatty acid, and carrying out polycondensation reaction using the ester compound as a monomer, a copolymer of the present invention represented by the following General Formula (1) is produced. 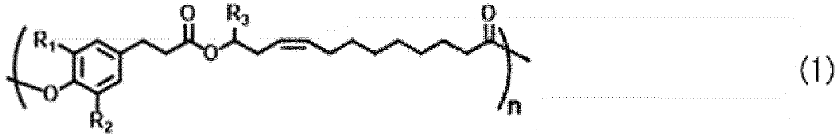 In the formula, the substituents R₁ and R₂ each represent a hydrogen atom or a methoxy group. n is an integer of 10 to 100, preferably 20 to 100. The substituent R₃ represents an alkyl group or an alkenyl group. The number of carbon atoms in the alkyl group or alkenyl group is 1 to 15, preferably 1 to 10. Examples of the substituent R₃ include alkyl groups such as C₆H₁₃.

Specific examples of the polyester compound obtained by the present invention are shown below as the structural formulae (4) to (10).

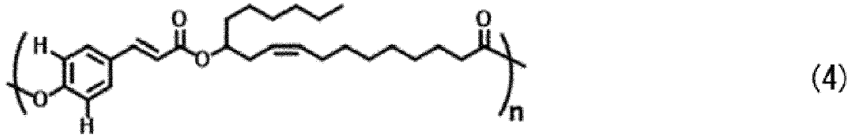

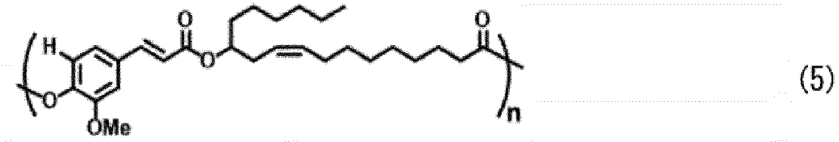

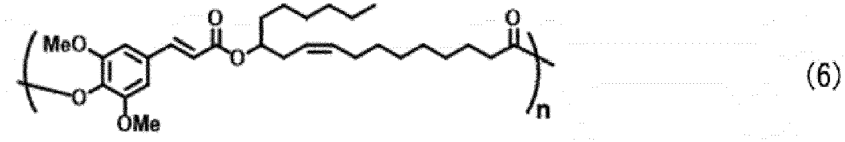

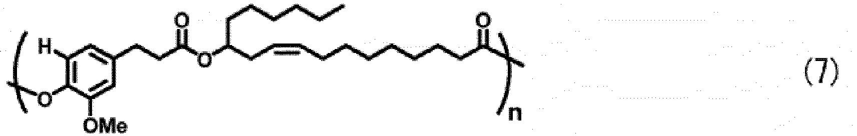

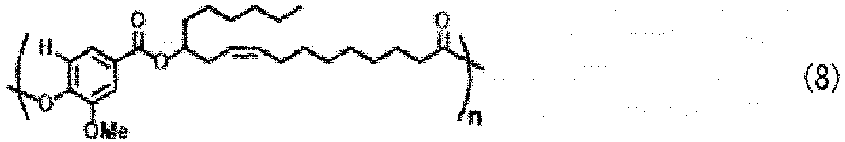

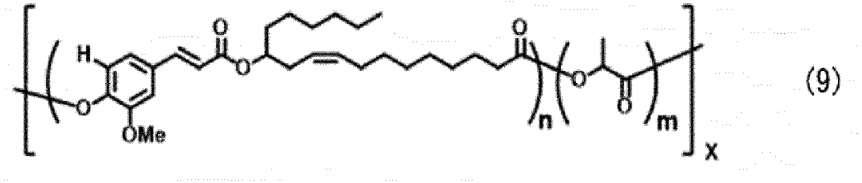

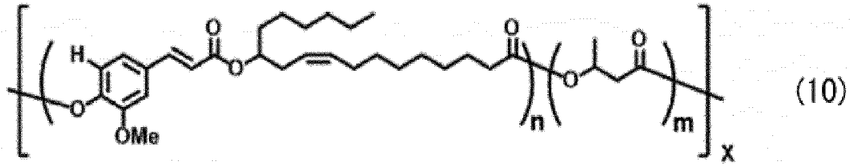

In these formulae, n, m, and X are each independently an integer of 10 to 100, preferably 20 to 100.

The novel polyester compound of the present invention is extremely useful as a rubber material or an adhesive material derived from a biomass. These compounds have regular structures, whose production is difficult for the random copolymerization method, which is a conventional standard means in the art.

The present invention is described below in more detail by way of Examples.

The production methods described below are merely examples for the purpose of helping understanding of the present invention, and the present invention is not limited to these.

### EXAMPLE 1

### [Production of Polyester Compound Represented by General Formula (4)]

In 20 mL of dichloromethane, 4.45 g of monomers (an ester compound composed of coumaric acid and ricinoleic acid) and 0.59 g of p-toluenesulfonic acid-4-dimethylaminopyridinium were dissolved. This solution was sufficiently cooled by immersion in a cooling medium at 0°C, and then vigorously stirred. To the solution, 2.3 mL of N,N-diisopropylcarbodiimide was added dropwise using a syringe. Thereafter, the reaction solution was removed from the cooling medium and allowed to warm to room temperature, followed by stirring the solution for 24 hours to perform polycondensation reaction. After the predetermined time has passed, 200 mL of methanol was added to the reaction solution. As a result, a white viscous substance precipitated. The resulting viscous substance was washed with methanol, and then dried by heating under vacuum (60°C, 12 hours), to obtain 3.21 g of a colorless highly viscous compound (number average molecular weight, 11,265).

### EXAMPLE 2

### [Production of Polyester Compound Represented by General Formula (5)]

In 20 mL of dichloromethane, 4.75 g of monomers (an ester compound composed of ferulic acid and ricinoleic acid) and 0.59 g of p-toluenesulfonic acid-4-dimethylaminopyridinium were dissolved. This solution was sufficiently cooled by immersion in a cooling medium at 0°C, and then vigorously stirred. To the solution, 2.3 mL of N,N-diisopropylcarbodiimide was added dropwise using a syringe. Thereafter, the reaction solution was removed from the cooling medium and allowed to warm to room temperature, followed by stirring the solution for 24 hours to perform polycondensation reaction. After the predetermined time has passed, 200 mL of methanol was added to the reaction solution. As a result, a white viscous substance precipitated. The resulting viscous substance was washed with methanol, and then dried by heating under vacuum (60°C, 12 hours), to obtain 3.23 g of a colorless highly viscous compound (number average molecular weight, 19,471).

### EXAMPLE 3

### [Production of Polyester Compound Represented by Formula (6)]

In 20 mL of dichloromethane, 5.05 g of monomers (an ester compound composed of sinapic acid and ricinoleic acid) and 0.59 g of p-toluenesulfonic acid-4-dimethylaminopyridinium were dissolved. This solution was sufficiently cooled by immersion in a cooling medium at 0°C, and then vigorously stirred. To the solution, 2.3 mL of N,N-diisopropylcarbodiimide was added dropwise using a syringe. Thereafter, the reaction solution was removed from the cooling medium and allowed to warm to room temperature, followed by stirring the solution for 24 hours to perform polycondensation reaction. After the predetermined time has passed, 200 mL of methanol was added to the reaction solution. As a result, a white viscous substance precipitated. The resulting viscous substance was washed with methanol, and then dried by heating under vacuum (60°C, 12 hours), to obtain 3.18 g of a colorless highly viscous compound (number average molecular weight, 21,297).

### EXAMPLE 4

### [Production of Polyester Compound Represented by Formula (7)]

In 1.0 mL of dichloromethane, 0.24 g of monomers (an ester compound composed of dihydroferulic acid and ricinoleic acid) and 0.03 g of p-toluenesulfonic acid-4-dimethylaminopyridinium were dissolved. This solution was sufficiently cooled by immersion in a cooling medium at 0°C, and then vigorously stirred. To the solution, 0.12 mL of N,N-diisopropylcarbodiimide was added dropwise using a syringe. Thereafter, the reaction solution was removed from the cooling medium and allowed to warm to room temperature, followed by stirring the solution for 24 hours to perform polycondensation reaction. After the predetermined time has passed, 5.0 mL of methanol was added to the reaction solution. As a result, a white viscous substance precipitated. The resulting viscous substance was washed with methanol, and then dried by heating under vacuum (60°C, 12 hours), to obtain 0.16 g of a colorless highly viscous compound (number average molecular weight, 10,987).

### EXAMPLE 5

### [Production of Polyester Compound Represented by Formula (8)]

In 1.0 mL of dichloromethane, 0.22 g of monomers (an ester compound composed of vanillic acid and ricinoleic acid) and 0.03 g of p-toluenesulfonic acid-4-dimethylaminopyridinium were dissolved. This solution was sufficiently cooled by immersion in a cooling medium at 0°C, and then vigorously stirred. To the solution, 0.12 mL of N,N-diisopropylcarbodiimide was added dropwise using a syringe. Thereafter, the reaction solution was removed from the cooling medium and allowed to warm to room temperature, followed by stirring the solution for 24 hours to perform polycondensation reaction. After the predetermined time has passed, 5.0 mL of methanol was added to the reaction solution. As a result, a white viscous substance precipitated. The resulting viscous substance was washed with methanol, and then dried by heating under vacuum (60°C, 12 hours), to obtain 0.21 g of a colorless highly viscous compound (number average molecular weight, 27,824).

### EXAMPLE 6

### [Production of Polyester Compound Represented by Formula (9)]

In 2.0 mL of dichloromethane, 0.47 g of monomers (an ester compound composed of ferulic acid and ricinoleic acid), 0.096 g of lactic acid, and 0.12 g of p-toluenesulfonic acid-4-dimethylaminopyridinium were dissolved. This solution was sufficiently cooled by immersion in a cooling medium at 0°C, and then vigorously stirred. To the solution, 0.46 mL of N,N-diisopropylcarbodiimide was added dropwise using a syringe. Thereafter, the reaction solution was removed from the cooling medium and allowed to warm to room temperature, followed by stirring the solution for 24 hours to perform polycondensation reaction. After the predetermined time has passed, 5.0 mL of methanol was added to the reaction solution. As a result, a white viscous substance precipitated. The resulting viscous substance was washed with methanol, and then dried by heating under vacuum (60°C, 12 hours), to obtain 0.47 g of a colorless highly viscous compound (number average molecular weight, 28,681).

### EXAMPLE 7

### [Production of Polyester Compound Represented by Formula (10)]

In 2.0 mL of dichloromethane, 0.47 g of monomers (an ester compound composed of ferulic acid and ricinoleic acid), 0.11 g of (R)-3-hydroxybutanoic acid, and 0.12 g of p-toluenesulfonic acid-4-dimethylaminopyridinium were dissolved. This solution was sufficiently cooled by immersion in a cooling medium at 0°C, and then vigorously stirred. To the solution, 0.46 mL of N,N-diisopropylcarbodiimide was added dropwise using a syringe. Thereafter, the reaction solution was removed from the cooling medium and allowed to warm to room temperature, followed by stirring the solution for 24 hours to perform polycondensation reaction. After the predetermined time has passed, 5.0 mL of methanol was added to the reaction solution. As a result, a white viscous substance precipitated. The resulting viscous substance was washed with methanol, and then dried by heating under vacuum (60°C, 12 hours), to obtain 0.18g of a colorless highly viscous compound (number average molecular weight, 9,988).

### [NMR Measurement Results]

The values described below are examples of NMR measurement results for the compounds of the present invention. The produced compounds (4) to (10) correspond to the General Formulae (4) to (10) shown above.

### Compound (4) (n=27)

¹H-NMR (CDCl₃, ppm)
0.86-0.89, 1.20-1.45, 1.59-1.63, 1.67-1.76, 2.03-2.06, 2.33-2.43, 2.51-2.55, 4.99-5.05, 5.36-5.53, 6.36-6.40, 7.09-7.12, 7.52-7.54, 7.62-7.66
¹³C-NMR (CDCl₃, ppm)
14.07, 22.59, 24.85, 25.39, 27.36, 29.05, 29.12, 29.19, 29.53, 31.74, 32.04, 33.71, 34.35, 74.23, 118.73, 122.11, 124.26, 129.12, 132.13, 132.67, 143.33, 152.14, 166.59, 171.93

Compound (1) was dried by heating under vacuum (60°C, 12 hours), to completely remove the solvent by distillation. As a result, it was found to be an elastic viscous compound. When the compound was attached to a glass plate, it showed strong adhesion. When the glass plate was inverted in this state, the subject kept adhering to the plate without falling off by itself. The subject was left to stand overnight (for about 17 hours) in this state, and then the condition of the subject was observed. As a result, neither dropping off nor peeling off of the subject was found.

### Compound (5) (n=43)

¹H-NMR (CDCl₃, ppm)
0.86-0.89, 1.20-1.45, 1.59-1.63, 1.70-1.78, 2.03-2.06, 2.33-2.43, 2.54-2.58, 3.84, 4.99-5.05, 5.36-5.53, 6.35-6.39, 7.02-7.12, 7.60-7.64
¹³C-NMR (CDCl₃, ppm)
14.07, 22.60, 24.97, 25.40, 27.38, 29.01, 29.20, 29.55, 31.75, 32.04, 33.71, 34.00, 55.89, 74.27, 111.18, 118.76, 121.22, 123.25, 124.22, 132.71, 133.37, 141.51, 143.77, 151.44, 166.55, 171.58

Compound (2) was dried by heating under vacuum (60°C, 12 hours), to completely remove the solvent by distillation. As a result, it was found to be an elastic viscous compound. When the compound was attached to a glass plate, it showed strong adhesion. When the glass plate was inverted in this state, the subject kept adhering to the plate without falling off by itself. The subject was left to stand overnight (for about 17 hours) in this state, and then the condition of the subject was observed. As a result, neither dropping off nor peeling off of the subject was found.

### Compound (6) (n=44)

¹H-NMR (CDCl₃, ppm)
0.86-0.89, 1.20-1.45, 1.59-1.63, 1.72-1.80, 2.03-2.06, 2.33-2.43, 2.58-2.62, 3.83, 4.99-5.05, 5.36-5.53, 6.35-6.39, 6.76, 7.57-7.61
¹³C-NMR (CDCl₃, ppm)
14.07, 22.60, 25.05, 25.39, 27.39, 28.97, 29.19, 29.54, 31.74, 32.03, 33.71, 33.83, 56.16, 74.28, 104.65, 118.82, 124.17, 130.45, 132.69, 132.74, 144.19, 152.45, 166.47, 171.28

Compound (3) was dried by heating under vacuum (60°C, 12 hours), to completely remove the solvent by distillation. As a result, it was found to be an elastic viscous compound. When the compound was attached to a glass plate, it showed strong adhesion. When the glass plate was inverted in this state, the subject kept adhering to the plate without falling off by itself. The subject was left to stand overnight (for about 17 hours) in this state, and then the condition of the subject was observed. As a result, neither dropping off nor peeling off of the subject was found.

### Compound (7) (n=24)

¹H-NMR (CDCl₃, ppm)
0.86-0.89, 1.20-1.41, 1.48-1.56, 1.70-1.79, 1.97-2.07, 2.20-2.35, 2.52-2.63, 2.88-2.96, 3.79, 4.86-4.93, 5.28-5.49, 6.75-6.80, 6.90-6.93
¹³C-NMR (CDCl₃, ppm)
14.02, 22.53, 24.96, 25.27, 27.29, 28.97, 29.08, 29.47, 30.91, 31.64, 31.88, 33.54, 33.95, 36.05, 55.72, 74.11, 112.48, 120.24, 122.56, 124.14, 132.57, 138.09, 139.39, 150.84, 171.88, 172.44

Compound (7) was dried by heating under vacuum (60°C, 12 hours), to completely remove the solvent by distillation. As a result, it was found to be an elastic viscous compound. When the compound was attached to a glass plate, it showed strong adhesion. When the glass plate was inverted in this state, the subject kept adhering to the plate without falling off by itself. The subject was left to stand overnight (for about 17 hours) in this state, and then the condition of the subject was observed. As a result, neither dropping off nor peeling off of the subject was found.

### Compound (8) (n=65)

¹H-NMR (CDCl₃, ppm)
0.85-0.88, 1.20-1.44, 1.60-1.80, 2.00-2.10, 2.36-2.50, 2.55-2.61, 3.87, 5.09-5.15, 5.37-5.52, 7.06-7.08, 7.65-6.67
¹³C-NMR (CDCl₃, ppm)
14.02, 22.53, 24.87, 25.33, 27.33, 28.95, 29.12, 29.47, 31.66, 31.99, 33.67, 33.93, 55.96, 74.92, 113.37, 122.42, 122.62, 124.09, 129.29, 132.70, 143.53, 150.97, 165.53, 171.34

Compound (8) was dried by heating under vacuum (60°C, 12 hours), to completely remove the solvent by distillation. As a result, it was found to be an elastic viscous compound. When the compound was attached to a glass plate, it showed strong adhesion. When the glass plate was inverted in this state, the subject kept adhering to the plate without falling off by itself. The subject was left to stand overnight (for about 17 hours) in this state, and then the condition of the subject was observed. As a result, neither dropping off nor peeling off of the subject was found.

### Compound (9) (Ferulic Acid - Ricinoleic Acid: Lactic Acid = 48:52)

¹H-NMR (CDCl₃, ppm)
0.86-0.89, 1.20-1.45, 1.59-1.80, 2.03-2.06, 2.33-2.43, 2.58-2.62, 3.83, 4.99-5.53, 6.35-6.39, 7.00-7.12, 7.59-7.64

Compound (9) was dried by heating under vacuum (60°C, 12 hours), to completely remove the solvent by distillation. As a result, it was found to be an elastic viscous compound. When the compound was attached to a glass plate, it showed strong adhesion. When the glass plate was inverted in this state, the subject kept adhering to the plate without falling off by itself. The subject was left to stand overnight (for about 17 hours) in this state, and then the condition of the subject was observed. As a result, neither dropping off nor peeling off of the subject was found.

### Compound (10) (Ferulic Acid - Ricinoleic Acid: (R)-3-Hydroxybutanoic Acid = 50:50)

¹H-NMR (CDCl₃, ppm)
0.86-0.89, 1.20-1.45, 1.59-1.80, 2.03-2.06, 2.21-2.68, 2.73-2.79, 2.89-2.95, 3.84, 4.99-5.03, 5.24-5.53, 6.35-6.39, 7.00-7.16, 7.59-7.64

Compound (10) was dried by heating under vacuum (60°C, 12 hours), to completely remove the solvent by distillation. As a result, it was found to be an elastic viscous compound. When the compound was attached to a glass plate, it showed strong adhesion. When the glass plate was inverted in this state, the subject kept adhering to the plate without falling off by itself. The subject was left to stand overnight (for about 17 hours) in this state, and then the condition of the subject was observed. As a result, neither dropping off nor peeling off of the subject was found.

## Claims

1. A polyester compound comprising a copolymer containing a hydroxycinnamic acid and a fatty acid that are alternately directly linked to each other.

2. The polyester compound according to claim 1, wherein the hydroxycinnamic acid is a compound represented by the following General Formula (2): 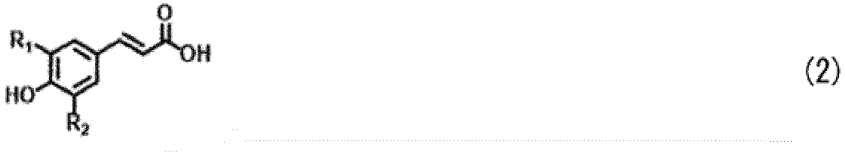 (wherein in the formula, the substituents R₁ and R₂ each represent a hydrogen atom or a methoxy group).

3. The polyester compound according to claim 1 or 2, wherein the fatty acid is a compound represented by the following General Formula (3): 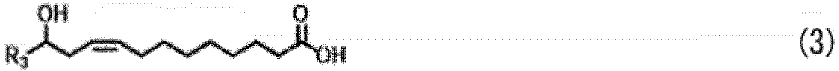 (wherein in the formula, the substituent R₃ represents an alkyl group or an alkenyl group).

4. The polyester compound according to any one of claims 1 to 3, wherein the copolymer is represented by the following General Formula (1): 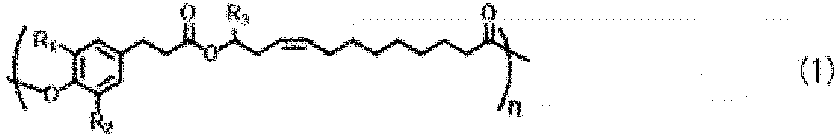 (wherein in the formula, the substituents R₁ and R₂ each represent a hydrogen atom or a methoxy group; the substituent R₃ represents an alkyl group or an alkenyl group; and n represents an integer of 10 to 100).

5. The polyester compound according to any one of claims 1 to 4, wherein the hydroxycinnamic acid and/or the fatty acid is/are produced by fermentation using a biomass as a raw material, produced by hydrolysis reaction using a biomass as a raw material, or produced by extraction using a biomass as a raw material.

6. The polyester compound according to any one of claims 1 to 5, further comprising a third component.
